# EUROPÄISCHE PATENTSCHRIFT

(11) **EP 0 710 111 B1**
(45) Veröffentlichungstag und Bekanntmachung des Hinweises auf die Patenterteilung: **19.09.2001**
(21) Anmeldenummer: 94916880.1
(22) Anmeldetag: 03.06.1994
(51) Int. Cl.: A61K 31/57, A61K 31/565

(54) **HORMONALES MITTEL ZUR THERAPIE DER AKNE UND DESSEN VERWENDUNG**
HORMONAL AGENT FOR THE TREATMENT OF ACNE AND ITS USE
AGENT HORMONAL POUR LA THERAPIE DE L'ACNE ET SON UTILISATION

(30) Priorität: 01.07.1993 DE 4321957
(43) Veröffentlichungstag der Anmeldung: 08.05.1996
(73) Patentinhaber: Ehrlich, Marika, Dr. med., D-55234 Framersheim (DE); Kuhl, Herbert, Prof. Dr., D-63741 Aschaffenburg (DE)
(72) Erfinder: Ehrlich, Marika, Dr. med., D-55234 Framersheim (DE); Kuhl, Herbert, Prof. Dr., D-63741 Aschaffenburg (DE)
(74) Vertreter: Goddar, Heinz J., Dr.
(86) Internationale Anmeldenummer: DE9400629
(87) Internationale Veröffentlichungsnummer: WO9501180

(56) Entgegenhaltungen:
- DE-A- 4 104 385
- CONTRACEPTION,, VOL. 42, NO. 2, PAGE(S) 225-234, 1990 FUGERE P et al 'CYPROTERONE ACETATE ETHYNYLESTRADIOL IN THE TREATMENT OF ACNE A COMPARATIVE DOSE-RESPONSE STUDY OF THE ESTROGEN COMPONENT'
- J ENDOCRINOL INVEST,, VOL. 10, NO. 3, PAGE(S) 237-239, 1987 PARR J H et al 'THE EFFECTS OF REVERSE SEQUENTIAL ANTI-ANDROGEN THERAPY CYPROTERONE ACETATE AND ETHYNYLESTRADIOL ON HEMATOLOGICAL PARAMETERS'

## Beschreibung

Die Erfindung betrifft ein hormonales Mittel zur Therapie der Akne, mit zwei in einer Verpackungseinheit räumlich getrennt konfektionierten, zur zeitlich sequentiellen oralen Verabreichung bestimmten Hormonkomponenten, die jeweils aus einer Anzahl räumlich getrennt und einzeln entnehmbar in der Verpackungseinheit untergebrachter Hormon-Tageseinheiten bestehen, wobei eine erste der Hormonkomponenten als hormonellen Wirkstoff im wesentlichen ausschließlich ein einen Anstieg der sexualhormonbindenden Globuline (SHBG) bewirkendes Östrogenpräparat, die zweite Hormonkomponente hingegen in Kombination ein Östrogen- und ein Antiandrogenpräparat enthält, sowie dessen Verwendung.

Seit langem ist bereits ein hormonales Mittel zur Therapie der Akne der eingangs genannten Art bekannt, welches als Kombinationspräparat für Fälle von Seborrhö, Akne und Alopezia Androgenetica sowie für leichtere Fälle von Hirsutismus zur Verfügung steht und als Antiandrogenpräparat 2 mg Cyproteronacetat enthält, während die erste und die zweite Hormonkomponente als Östrogenpräparat 35 *µ*g Ethinylestradiol aufweisen. In seiner Wirkung ähnlich ist ein Kombinationspräparat mit 2 mg Chlormadinonacetat und 50 *µ*g Mestranol.

Bei den bekannten hormonalen Mitteln zur Therapie der Akne, wie sie vorstehend beschrieben sind, handelt es sich um Kombinationspräparate, die neben der aknebekämpfenden Wirkung auch als Ovulationshemmer wirksam sind. Sofern die gewünschte Zyklusdauer 28 Tage beträgt, werden diese Mittel 21 Tage lang verabreicht, wobei sich an die betreffenden 21 Tageseinheiten eine siebentägige Pause anschließt, in der keine Hormonverabreichung erfolgt und im Anschluß an die es zu einer die natürliche Monatsblutung simulierenden Entzugsblutung kommt.

Das eingangs genannte Mittel hat sich durchaus bewährt, allerdings hat es sich als durchaus wünschenswert herausgestellt, die Akne noch besser bekämpfen zu können.

Andererseits ist aus der DE-PS 41 04 385 ein ovulationshemmendes Mittel zur hormonalen Kontrazeption mit zwei in einer Verpackungseinheit räumlich getrennt konfektionierten, zur zeitlich sequentiellen oralen Verabreichung bestimmten Hormonkomponenten, die jeweils aus einer Anzahl räumlich getrennt und einzeln entnehmbar in der Verpackungseinheit untergebrachter Hormontageseinheiten bestehen, wobei eine erste der Hormonkomponenten als hormonellen Wirkstoff im wesentlichen ausschließlich ein eine Störung der Follikelreifung bewirkendes Östrogenpräparat, die zweite Hormonkomponente hingegen in Kombination ein Östrogenund in mindestens zur Ovulationshemmung ausreichender Dosierung ein Gestagenpräparat enthält, bekannt, bei dem die Gesamtzahl der Hormon-Tageseinheiten gleich der Gesamtzahl der Tage des gewünschten Zyklus ist, die erste Hormonkomponente 5 bis 14 und die zweite Hormonkomponente 23 bis 14 Tageseinheiten umfaßt, wobei die Anzahl der Tageseinheiten der ersten Hormonkomponente geringer als die Anzahl der Tageseinheiten der zweiten Hormonkomponente ist.

Bei diesem bekannten ovulationshemmenden Mittel kann das Östrogenpräparate beispielsweise Ethinylestradiol und das Gestagen- oder Antiandrogenpräparat Cyproteronacetat oder Chlormadinonacetat aufweisen.

Charakteristisch ist für das vorstehend beschriebene ovulationshemmende Mittel, daß die Verabreichung ohne Einnahmepause mit konstanter Östrogendosierung erfolgt, wodurch ein gleichmäßiger Östrogenspiegel aufrechterhalten wird.

Der Erfindung liegt die Aufgabe zugrunde, das gattungsgemäße Mittel zur Therapie der Akne dahingehend weiterzubilden, daß eine verbesserte Aknetherapie ermöglicht wird.

Erfindungsgemäß wird diese Aufgabe dadurch gelöst, daß die Gesamtzahl der Tageseinheiten gleich der Gesamtzahl der Tage des gewünschten Zyklus ist; daß die erste Hormonkomponente 5 bis 14 und und die zweite Hormonkomponente 23 bis 14 Tageseinheiten umfaßt; und daß die Anzahl der Tageseinheiten der ersten Hormonkomponente geringer als die Anzahl der Tageseinheiten der zweiten Hormonkomponente ist.

Dabei kann vorgesehen sein, daß mindestens eines der Östrogenpräparate mindestens einen Bestandteil aus der Ethinylestradiol, Mestranol, andere synthetische Östrogene sowie mindestens einen der vorgenannten Hormonbestandteile nach Einnahme rasch abspaltende Hormonverbindungen umfassenden Gruppe aufweist.

Die Erfindung sieht auch vor, daß die Östrogenpräparate der ersten und der zweiten Hormonkomponente ihrer Art und/oder ihrer wirksamen Dosierung nach identisch sind.

Nach der Erfindung kann auch vorgesehen sein, daß die erste und die zweite Hormonkomponente als Östrogenpräparat Ethinylestradiol mit einer Konzentration von 15 - 35 *µ*g pro Tageseinheit aufweisen.

Weiterhin schlägt die Erfindung vor, daß das Antiandrogenpräparat Cyproteronacetat aufweist.

Nach der Erfindung kann auch vorgesehen sein, daß das Antiandrogenpräparat pro Tageseinheit ca. 1 - 2 mg Cyproteronacetat aufweist.

Eine weitere Ausführungsform der Erfindung ist dadurch gekennzeichnet, daß das Antiandrogenpräparat Chlormadinonacetat aufweist.

Die Erfindung schlägt auch vor, daß das Antiandrogenpräparat pro Tageseinheit ca. 1 - 3 mg Chlormadinonacetat aufweist.

Die Erfindung schlägt dabei ggf. auch vor, daß das Anti-antrogenpräparat Dienogest aufweist.

Auch kann vorgesehen sein, daß das Antiandrogenpräparat pro Tageseinheit ca. 1 - 3 mg Dienogest aufweist.

Nach der Erfindung kann auch vorgesehen sein, daß die Gesamtzahl der Tageseinheiten 28 beträgt.

Die Erfindung sieht gegebenenfalls auch vor, daß die erste Hormonkomponente höchstens 10 Tageseinheiten umfaßt.

Die Erfindung schlägt auch vor, daß die erste Hormonkomponente 7 und die zweite Hormonkomponente 21 Tageseinheiten umfaßt.

Schließlich lehrt die Erfindung auch die Verwendung des hormonalen Mittels nach der Erfindung zur Aknetherapie.

Der Erfindung liegt die überraschende Erkenntnis zugrunde, daß es gelingt, eine verbesserte Aknetherapie zu ermöglichen, wenn entsprechend dem ovulationshemmenden Mittel nach DE-PS 41 04 385 auf jedwede Einnahmepause verzichtet und über den gesamten Zyklus von z. B. von 28 Tagen ein gleichmäßiger Östrogenspiegel, insbesondere auf der Basis von Ethinylestradiol, aufrechterhalten wird. Hierdurch wird eine im wesentlichen konstant erhöhte Konzentration des SHBG's bewirkt, die das freie Testosteron abfängt, wodurch die Akne günstig beeinflußt wird.

Natürlich tritt auch bei dem Mittel nach der Erfindung eine ovulationshemmende Wirkung entsprechend dem ovulationshemmenden Mittel nach DE-OS 41 04 385 auf, die ggf. erwünscht sein kann. Auch die anderen, in der DE-PS 41 04 385 beschriebenen Vorteile der Aufrechterhaltung eines gleichmäßigen Östrogenspiegels, der durch die Vermeidung jedweder Einnahmepause bewirkt wird, werden in günstiger Weise bei der Verabreichung des Mittels nach der Erfindung erzielt.

Weitere Merkmale der Erfindung ergeben sich aus der nachfolgenden Beschreibung, in der Ausführungsbeispiele erläutert sind.

### Beispiel 1:

Zur Aknetherapie wurde ein Kombinationspräparat verwendet, welches 7 Tageseinheiten mit jeweils 20 *µ*g Ethinylestradiol sowie 21 Tageseinheiten mit jeweils 20 *µ*g Ethyinylestradiol und 2 mg Cyproteronacetat enthielt. Das Mittel wurde über ein Jahr verabreicht und zeige eine sehr gute aknebekämpfende Wirkung.

### Beispiel 2:

Zur Aknetherapie wurde ein Kombinationspräparat verwendet, welches 7 Tageseinheiten mit jeweils 20 *µ*g Ethinylestradiol und 21 Tageseinheiten mit je 20 *µ*g Ethinylestradiol und 2 mg Chlormadinonacetat aufwies. Die Wirkungsweise entsprach derjenigen von Beispiel 1.

### Beispiel 3:

Als Präparat zur Aknetherapie wurde ein Kombinationspräparat verwendet, welches 7 Tageseinheiten mit jeweils 20 *µ* Ethinylestradiol und 21 Tageseinheiten mit je 20 *µ*g Ethinylestradiol und 2 mg Dienogest aufwies. Die Wirkungsweise entsprach derjenigen von Beispiel 1 und Beispiel 2.

Die in der vorstehenden Beschreibung und in den Ansprüchen offenbarten Merkmale der Erfindung können sowohl einzeln als auch in beliebiger Kombination für die Verwirklichung der Erfindung in ihren verschiedenen Ausführungsformen wesentlich sein.

## Patentansprüche

1. Hormonales Mittel zur Therapie der Akne, mit zwei in einer Verpackungseinheit räumlich getrennt konfektionierten, zur zeitlich sequentiellen oralen Verabreichung bestimmten Hormonkomponenten, die jeweils aus einer Anzahl räumlich getrennt und einzeln entnehmbar in der Verpackungseinheit untergebrachter Hormon-Tageseinheiten bestehen, wobei eine erste der Hormonkomponenten als hormonellen Wirkstoff im wesentlichen ausschließlich ein einen Anstieg der sexualhormonbindenden Globuline (SHBG) bewirkendes Östrogenpräparat, die zweite Hormonkomponente hingegen in Kombination ein Östrogen- und ein Antiandrogenpräparat enthält, **dadurch gekennzeichnet, daß** die Gesamtzahl der Tageseinheiten gleich der Gesamtzahl der Tage des gewünschten Zyklus ist; daß die erste Hormonkomponente 5 bis 14 und und die zweite Hormonkomponente 23 bis 14 Tageseinheiten umfaßt; und daß die Anzahl der Tageseinheiten der ersten Hormonkomponente geringer als die Anzahl der Tageseinheiten der zweiten Hormonkomponente ist.

2. Mittel nach Anspruch 1, **dadurch gekennzeichnet, daß** mindestens eines der Östrogenpräparate mindestens einen Bestandteil aus der Ethinylestradiol, Mestranol, andere synthetische Östrogene sowie mindestens einen der vorgenannten Hormonbestandteile nach Einnahme rasch abspaltende Hormonverbindungen umfassenden Gruppe aufweist.

3. Mittel nach einem der vorangehenden Ansprüche, **dadurch gekennzeichnet, daß** die Östrogenpräparate der ersten und der zweiten Hormonkomponente ihrer Art und/oder ihrer wirksamen Dosierung nach identisch sind.

4. Mittel nach Anspruch 3, **dadurch gekennzeichnet, daß** die erste und die zweite Hormonkomponente als Östrogenpräparat Ethinylestradiol mit einer Konzentration von 15 - 35 *µ*g pro Tageseinheit aufweisen.

5. Mittel nach einem der vorangehenden Ansprüche, **dadurch gekennzeichnet, daß** das Antiandrogenpräparat Cyproteronacetat aufweist.

6. Mittel nach Anspruch 5, **dadurch gekennzeichnet, daß** das Antiandrogenpräparat pro Tageseinheit ca. 1 - 2 mg Cyproteronacetat aufweist.

7. Mittel nach einem der Ansprüche 1 bis 4, **dadurch gekennzeichnet, daß** das Antiandrogenpräparat Chlormadinonacetat aufweist.

8. Mittel nach Anspruch 7, **dadurch gekennzeichnet, daß** das Antiandrogenpräparat pro Tageseinheit ca. 1 - 3 mg Chlormadinonacetat aufweist.

9. Mittel nach einem der Ansprüche 1 bis 4, **dadurch gekennzeichnet, daß** das Antiandrogenpräparat Dienogest aufweist.

10. Mittel nach Anspruch 9, **dadurch gekennzeichnet, daß** das Antiandrogenpreparat pro Tageseinheit ca. 1 - 3 mg Dienogest aufweist.

11. Mittel nach einem der vorangehenden Ansprüche, **dadurch gekennzeichnet, daß** die Gesamtzahl der Tageseinheiten 28 beträgt.

12. Mittel nach einem der vorangehenden Ansprüche, **dadurch gekennzeichnet, daß** die erste Hormonkomponente höchstens 10 Tageseinheiten umfaßt.

13. Mittel nach Anspruch 9 oder nach den Ansprüchen 9 und 10, **dadurch gekennzeichnet, daß** die erste Hormonkomponente 7 und die zweite Hormonkomponente 21 Tageseinheiten umfaßt.

14. Verwendung des Mittels nach einem der vorangehenden Ansprüche für die Herstellung eines Medikaments zur Behandlung von Akne.

## Claims

1. A hormonal agent for treatment of acne, comprising two hormone components packed at separate places in a packaging unit for administration one after the other, each containing a number of spatially separate hormone daily units individually removable from the packaging unit, a first hormone component containing a hormonal active substance consisting substantially exclusively of an oestrogen preparation which increases the sexual hormone-binding globulins (SHBG) whereas the second hormone component contains an oestrogen and an anti-androgen preparation in combination, **characterised in that** the total number of daily units is equal to the total number of days in the desired cycle; the first hormone component comprises 5 to 14 daily units and the second hormone component 23 to 14 daily units; and the number of daily units of the first hormone component is less than the number of daily units of the second hormone component.

2. An agent according to claim 1, **characterised in that** at least one of the oestrogen preparations contains at least one component chosen from ethinyl estradiol, mestranol, other synthetic oestrogens and at least [one] group comprising hormone compounds which rapidly split off one of the said hormone components after ingestion.

3. An agent according to any of the preceding claims, **characterised in that** the oestrogen preparations in the first and the second hormone component are identical in kind and/or in effective dosage.

4. An agent according to claim 3, **characterised in that** the first and the second hormone component comprise an oestrogen preparation in the form of ethinyl estradiol in a concentration of 15 - 35 µg per daily unit.

5. An agent according to any of the preceding claims, **characterised in that** the anti-androgen preparation comprises cyproterone acetate.

6. An agent according to claim 5, **characterised in that** the anti-androgen preparation contains about 1 to 2 mg cyproterone acetate per daily unit.

7. An agent according to any of claims 1 to 4, **characterised in that** the anti-androgen preparation comprises chlormadinone acetate.

8. An agent according to claim 7, **characterised in that** the anti-androgen preparation contains about 1 - 3 mg chlormadinone acetate per daily unit.

9. An agent according to any of claims 1 to 4, **characterised in that** the anti-androgen preparation comprises dienogest.

10. An agent according to claim 9, **characterised in that** the anti-androgen preparation contains about 1 - 3 mg dienogest per daily unit.

11. An agent according to any of the preceding claims, **characterised in that** the total number of daily units is 28.

12. An agent according to any of the preceding claims, **characterised in that** the first hormone component contains not more than 10 daily units.

13. An agent according to claim 9 or claims 9 and 10, **characterised in that** the first hormone component contains 7 daily units and the second contains 21 daily units.

14. Use of the agent according to any of the preceding claims for producing a drug for treatment of acne.

## Revendications

1. Agent hormonal pour la thérapie de l'acné avec deux composants hormonaux, déterminés pour l'administration orale temporellement séquentielle, composants confectionnés de façon spatialement séparée dans un ensemble d'emballage, chacun constitué d'une pluralité d'unités hormonales quotidiennes, logées dans l'ensemble d'emballage de façon séparée spatialement et susceptible d'être prélevées individuellement, un premier des composants hormonaux contenant, à titre de principe actif hormonal, essentiellement exclusivement une préparation oestrogénique provoquant une augmentation de la globuline liant l'hormone sexuelle (SHBG), alors que, par contre, l'autre composant hormonal contient, en combinaison, une préparation oestrogénique et une préparation antiandrogénique, **caractérisé en ce que** le nombre total des unités quotidiennes est identique au nombre global des jours du cycle souhaité; en ce que le premier composant hormonal comprend de 5 à 14 unités quotidiennes et le deuxième composant hormonal comprend de 23 à 14 unités quotidiennes; et en ce que le nombre d'unités quotidiennes du premier composant hormonal est inférieur au nombre d'unités quotidiennes du deuxième composant hormonal.

2. Agent selon la revendication 1, **caractérisé en ce qu'**au moins l'une des préparations oestrogéniques comprend au moins un constituant choisi dans le groupe comprenant l'éthinylestradiol, le mestranol, d'autres oestrogènes synthétiques, et au moins l'un des composants hormonaux précités comprenant des liaisons hormonales se dissociant rapidement après la prise.

3. Agent selon l'une des revendications précédentes, **caractérisé en ce que** la préparation oestrogénique du premier et du deuxième composant hormonal est de type et/ou de dosage actif identique.

4. Agent selon la revendication 3, **caractérisé en ce que** le premier et le deuxième composants hormonaux présentent, à titre de préparation oestrogénique, de l'éthinylestradiol en une concentration de 15 à 35 µg par unité quotidienne.

5. Agent selon l'une des revendications précédentes, **caractérisé en ce que** la préparation antiandrogénique comprend de l'acétate de cyprotérone.

6. Agent selon la revendication 5, **caractérisé en ce que** la préparation antiandrogénique comprend environ 1 à 2 mg de cyprotérone par unité quotidienne.

7. Agent selon l'une des revendications 1 à 4, **caractérisé en ce que** la préparation antiandrogénique comprend de l'acétate de chlormadinone.

8. Agent selon la revendication 7, **caractérisé en ce que** la préparation antiandrogénique comprend environ 1 à 3 mg d'acétate de chlormadinone par unité quotidienne.

9. Agent selon l'une des revendications 1 à 4, **caractérisé en ce que** la préparation antiandrogénique comprend du Dienogest.

10. Agent selon la revendication 9, **caractérisé en ce que** la préparation antiandrogénique comprend environ 1 à 3 mg de Dienogest par unité quotidienne.

11. Agent selon l'une des revendications précédentes, **caractérisé en ce que** le nombre total d'unités quotidiennes est de 28.

12. Agent selon l'une des revendications précédentes, **caractérisé en ce que** le premier composant hormonal comprend au maximum 10 unités quotidiennes.

13. Agent selon la revendication 9 ou selon les revendications 9 et 10, **caractérisé en ce que** le premier composant hormonal comprend 7 unités quotidiennes et le deuxième composant hormonal comprend 21 unités quotidiennes.

14. Utilisation de l'agent selon l'une des revendications précédentes, pour la préparation d'un médicament pour traitement de l'acné.
